Europäisches Patentamt

European Patent Office

Office européen des brevets

(11) Publication number: **0 207 516**
**A1**

(12) **EUROPEAN PATENT APPLICATION**

(21) Application number: 86109046.2

(22) Date of filing: 03.07.86

(51) Int. Cl.⁴: **A23L 1/236** , **A23G 3/30** , **A61K 7/16**

(30) Priority: 03.07.85 US 752229

(43) Date of publication of application:
07.01.87 Bulletin 87/02

(84) Designated Contracting States:
AT BE CH DE FR GB IT LI LU NL SE

(71) Applicant: GENERAL FOODS CORPORATION
250 North Street
White Plains, N.Y. 10625(US)

(72) Inventor: Palmer, Marcia D.
2 April Lane
Nanuet N.Y.(US)
Inventor: Hickernell, Gary Lee
12 Terrich Court
Ossining N.Y.(US)
Inventor: Zanno, Paul R.
2 April Lane
Nanuet N.Y.(US)

(74) Representative: Eitle, Werner, Dipl.-Ing. et al
Hoffmann, Eitle & Partner Patentanwälte
Arabellastrasse 4
D-8000 München 81(DE)

(54) **An ingestible product containing a sweetness inhibitor and process for inhibiting sweetness in an ingestible product.**

(57) A sweetness inhibited ingestible product and a process for inhibiting sweetness is disclosed. In this product and process an effective sweetness inhibiting amount of a compound selected from the group consisting of an alkali metal hexyl sulfate, an alkaline earth metal hexyl sulfate, an alkali metal heptyl sulfate, an alkaline earth metal heptyl sulfate and mixtures thereof is added to an ingestible composition to which a sweetener has been added.

# An ingestible product containing a sweetness inhibitor and process for inhibiting sweetness in an ingestible product

The present invention is directed to a sweetness modifier. More particularly, the present invention is directed to a sweetness inhibitor, said sweetness inhibitor comprising a sweetness inhibiting amount of a compound selected from the group consisting of an alkali metal hexyl sulfate, an alkaline earth metal hexyl sulfate, an alkali metal heptyl sulfate, an alkaline earth metal heptyl sulfate and mixtures thereof.

Sweetness is one of the primary taste cravings of both animals and humans. Thus, the utilization of sweetening agents in foods in order to satisfy this sensory desire is well established. However, oftentimes sweetening agents are used in foods to provide functions other than sweetening. For example, sweetening agents can be utilized in foods as fillers, bulking agents, antimicrobial agents, freezing point depressants, stabilizers and the like.

In many instances, the utilization of a sweetening agent for purposes other than sweetening results in an excessively sweet taste. In those instances a modification of the formulation to reduce the sweetener level is required. This modification must, of course, occur without the concurrent reduction in the amount of sweetener utilized. Obviously, the sweetness level was incorporated to provide an additional function. Decrease in the sweetener will adversely effect this requirement.

One solution to this problem, known in the art, is the addition of a bitter or an acidic ingredient to the ingestible product to reduce sweetness perception. This solution has met with very little success in that the resultant ingestible product has an undesirable bitter or acidic taste.

Accordingly, in view of the above remarks it becomes readily apparent that it would be highly desirable to provide a sweetness inhibiting agent which, when added to ingestible compositions which are sweetened with naturally occurring, artificial or combinations of natural and artificial sweeteners, greatly reduce or eliminate unwanted or unpleasant sweet tastes thereby increasing the palatability of said sweet compositions.

Alkyl sulfates and salts thereof are known in the art. However, their utility has generally been limited to detergents and surfactants. Indeed, use of alkyl sulfates as detergents and surfactants is usually restricted to alkyls of at least 10 to 12 carbon atoms. The use of alkyl sulfates salts of any number of carbon atoms as sweetness inhibitors is unknown in the art.

It has now been discovered that alkali metal and alkaline earth metal salts of hexyl sulfate and heptyl sulfate, provide extraordinary inhibition of sweetness without alteration of the level of sweetener actually present in an ingestible product.

In accordance with the instant invention a process is provided for inhibiting sweetness in ingestible products which comprises adding an effective sweetness inhibiting amount of a compound selected from the group consisting of an alkali metal hexyl sulfate, an alkaline earth metal hexyl sulfate, an alkali metal heptyl sulfate, an alkaline earth metal heptyl sulfate and mixtures thereof to a sweetened ingestible composition.

The instant invention is directed to sweetness inhibition. In particular, the present invention is directed to the addition of an effective sweetness inhibiting amount of a sweetness inhibitor added to a sweetened ingestible product. This addition decreases sweetness perception.

The sweetness inhibitors within the contemplation of the present invention include alkali metal hexyl sulfates, alkaline earth metal hexyl sulfates, alkali metal heptyl sulfates, alkaline earth metal heptyl sulfates and mixtures thereof.

More preferably, the sweetness inhibitors of the present invention are sodium hexyl sulfate, potassium hexyl sulfate, calcium hexyl sulfate, magnesium hexyl sulfate, lithium hexyl sulfate, sodium heptyl sulfate, potassium heptyl sulfate, calcium heptyl sulfate, magnesium heptyl sulfate, lithium heptyl sulfate and mixtures thereof.

Still more preferably, the sweetness inhibitors of the instant invention are sodium hexyl sulfate, potassium hexyl sulfate, sodium heptyl sulfate, potassium heptyl sulfate and mixtures thereof.

Most preferably, the sweetness inhibitors of this invention are sodium hexyl sulfate, sodium heptyl sulfate and mixtures thereof.

The sweetness inhibitor of the present invention may be used in conjunction with any of a number of known natural and/or artificial sweeteners including, for example, sucrose, fructose, corn syrup solids, high fructose corn syrup, dextrose, xylitol, sorbitol, mannitol, acesulfam, thaumatin, invert sugar, saccharin, cyclamate, dihydrochalcone, hydrogenated glucose syrups, aspartame (L-aspartyl-L-phenylalanine methyl ester) and other dipeptides, glycyrrhizin, stevioside and the like.

Typical ingestible compositions in which the sweetness inhibitors of the present invention may be used to produce the ingestible products of this invention are, for example, beverages, (including soft drinks, carbonated beverages, ready to mix beverages and the like), infused foods (e.g. fruits and vegetables), sauces, condiments, salad dressings, fruit juices, syrups, desserts (including puddings, gelatin, baked goods and frozen desserts, such as ice creams and sherbets), icings and fillings, soft frozen products (such as soft frozen creams, soft frozen ice creams and yogurts, soft frozen toppings, such as diary or non-dairy whipped toppings), confections, toothpaste, mouthwashes, chewing gum, intermediate moisture foods, (e.g. rice and dog foods) and the like.

It is an aspect of the present invention that the sweetness inhibitors of the present invention eliminate perceived sweetness without substantially contributing a bitter, salty or sour taste of their own. That is, the inhibiting of sweet taste of a food product by the sweetness inhibitors of the present invention is not due to any taste inparted by the compound(s) which constitutes the sweetness inhibitor(s). Rather, sweetness inhibition is due to an unexpected and highly surprising effect which results when the sweetness inhibitor is combined with a sweetener in the food product and tasted by the consumer.

Accordingly, the discovery of the present invention resides in the utilization of very small quantities of the inhibiting compounds of this invention which reduce or even eliminate undesirable sweet and/or lingering aftertastes of sweetness imparted by ingestible products containing natural and/or artificial sweeteners without effecting the other desirable tastes and properties of the ingestible foodstuff or pharmaceutical product.

The versatility of the inhibitor compound of the present invention in the formulation of food products and pharmaceutical preparations is manifested in several ways. For example, the inhibitors can be added to an undesirably sweet product, for example, an overly sweet soft frozen product or infused vegetable product to reduce or eliminate the undesirable sweet taste but maintain the necessary soft, frozen or infusion properties of the product. On the other hand, the inhibitors can be incorporated into products in conjunction with added amounts of sweetener in order to form novel soft frozen products.

In order to achieve the inhibiting results of the present invention, the sweetness inhibiting compounds described above are generally added to a foodstuff or pharmaceutical product containing a sweetener at a level which is effective to inhibit sweetness perception of that product. More particularly, it is found that the results provided by the present invention occur when the inhibiting agent is added to the product (containing a sweetener) in an amount in the range of from about 0.005 to about 1.0 percent by weight. It is noted that greater concentrations of inhibitor are operable but not practical. Preferred concentrations of the sweetness inhibitors of the present invention are in the range of from about 0.05 percent to about 0.5 percent by weight. Most preferably, the sweetness inhibitor of this invention is present in a concentration of from about 0.1 percent to about 0.5 percent by weight. It is emphasized that the aboverecited concentrations are based on the total weight of the foodstuff or pharmaceutical product.

The sweetness content of foodstuffs and pharmaceuticals to which the inhibitors of the present invention may be added to inhibit sweetness, in general, may be in the range of between 1 percent to about 50 percent sucrose equivalency. Generally, the inhibiting effect provided by the compounds of the present invention is experienced over a wide range of acidity, e.g. pH of 2 to 10, preferably a pH of 3 to 7.5, in buffered or unbuffered formulations.

A reduction of perceived sweetness of between 1 % and 100% is achieved depending upon the particular ingestible product, the amount and kind of sweetener contained therein and concomitant amount of inhibitor employed.

The following examples are given to illustrate the instant invention. Since these examples are given for illustrative purposes only they should not be interpreted as limiting the invention in any way.

EXAMPLE 1

The Test Procedure

A taste panel was assembled. Members of the panel sipped, but did not swallow, various aqueous solutions. After tasting a sample, panel members rinsed with water to remove any residual taste sensation before tasting the next sample. The panel members matched the sweetness of each of the aqueous solutions presented to them with sucrose standards that ranged in concentration from 1% to 25% by weight. The panel members were not informed of the identity of the aqueous solutions. Upon correlation of the ratings, sweetness inhibiting effect of the inhibitors was statistically calculated.

EXAMPLE 2

Testing of Hexyl Sodium Sulfate

A series of aqueous solutions of sucrose with and without the addition of hexyl sodium sulfate were prepared. It is noted that among the "sucrose solutions" without hexyl sodium sulfate was the limiting case where the sucrose concentration was zero.

These samples were tested by the tasting panel in accordance with the procedure enumerated in Example 1. Upon correlation of the sweetness ratings reported by panel members, sweetness inhibition was manifested in terms of equivalent sucrose concentration. That is, the sweetness inhibiting effect of hexyl sodium sulfate was reported as the decrease in sweetness from a sweetness of a 5, 10 or 25 percent by weight aqueous sucrose solution. For example, a 5 percent sucrose solution to which 0.1 percent by weight hexyl sodium sulfate was added obtained the same statistical average sweetness rating from the tasting panel as that obtained by a 3.3 percent by weight aqueous sucrose solution.

Complete results of this test are reported below in Table I. It is noted that all test samples were carefully controlled in terms of their acidity. Thus, each solution includes a report of its acidity. Comparisons were based on samples having the same acidity level, that is, the same pH.

### TABLE I

| Conc. of Hexyl Sodium Sulfate, % by weight | Sucrose Conc. of Aqueous Sol'n to which Sulfate was added, % by wt. | Equiv. Aqueous Sucrose Sol'n Concen., % by wt. | pH |
|---|---|---|---|
| 0.1 | 5 | 3.3 | 7 |
| 0.2 | 5 | 1.3 | 7 |
| 0.3 | 5 | <1 | 7 |
| 0.5 | 5 | <1 | 7 |
| 1.0 | 5 | <1 | 7 |
| 0.3 | 10 | 3 | 7 |
| 0.5 | 10 | 1 | 7 |
| 0.3 | 10 | 0 | 3 |
| 0.2 | 25 | 13 | 7 |
| 0.4 | 25 | 8 | 7 |

EXAMPLE 3

Testing of Heptyl Sodium Sulfate

The procedure of Example 2 was repeated except heptyl sodium sulfate, instead of hexyl sodium sulfate, was utilized as the sweetness inhibitor. The results of this example are summarized in Table II below.

## TABLE II

| Conc. of Heptyl Sodium Sulfate % by weight | Sucrose Conc. of Aqueous Sol'n to which Sulfate was added, % by wt. | Equiv. Aqueous Sucrose Sol'n Concen., % by wt. | pH |
|---|---|---|---|
| 0.1 | 5 | 1.5 | 7 |
| 0.2 | 5 | <1 | 7 |
| 0.3 | 5 | 0 | 7 |
| 0.1 | 10 | 4 | 7 |
| 0.2 | 10 | 2.5 | 7 |
| 0.3 | 10 | <1 | 7 |
| 0.2 | 25 | 7.5 | 7 |
| 0.4 | 25 | <5 | 7 |

## DISCUSSION OF RESULTS

Tables I and II, summarizing the data of Examples 2 and 3, respectively, establish the sweetness inhibiting effect of the sweetness inhibitors of the present invention. Under neutral conditions a minimum of 33 percent up to a maximum of 90 percent or greater decrease in sweetness perception was obtained when hexyl sodium sulfate was employed as the sweetness inhibitor. Total sweetness inhibition, that is, 100% decrease in sweetness perception, was obtained in a test of an acidic solution of hexyl sodium sulfate.

Similar results were obtained utilizing heptyl sodium sulfate as the sweetness inhibitor. Indeed, this compound provided even greater sweetness inhibition. At neutral pH inhibition of 50 percent up to and including a maximum of 100 percent sweetness inhibition was noticed.

The above preferred embodiments and examples are provided to illustrate the scope and the spirit of the instant invention. These embodiments and examples will make apparent, to those skilled in the art, other embodiments and examples within the scope and spirit of this invention. These other embodiments and examples are within the contemplation of the present invention. Therefore, the scope of the instant invention should be limited only by the appended claims.

## Claims

1. An ingestible product comprising an ingestible composition, a sweetener and sweetness inhibitor, said inhibitor selected from the group consisting of an alkali metal hexyl sulfate, an alkali earth metal heptyl sulfate, an alkaline earth metal heptyl sulfate and mixtures thereof.

2. An ingestible product in accordance with Claim 1 wherein said inhibitor is selected from the group consisting of sodium hexyl sulfate, potassium hexyl sulfate, calcium hexyl sulfate, magnesium hexyl sulfate, lithium hexyl sulfate, sodium heptyl sulfate, potassium heptyl sulfate, calcium heptyl sulfate, magnesium heptyl sulfate, lithium heptyl sulfate and mixtures thereof.

3. An ingestible product in accordance with any of Claims 1 or 2 wherein said inhibitor is selected from the group consisting of sodium hexyl sulfate, potassium hexyl sulfate, sodium heptyl sulfate, potassium heptyl sulfate and mixtures thereof.

4. An ingestible product in accordance with any of Claims 1 to 3 wherein said sweetness inhibitor is present in a concentration in the range of between about 0.005 and 1.0 percent by weight, based on the total weight of said ingestible product.

5. An ingestible product in accordance with any of Claims 1-3 wherein said sweetness inhibitor is present in a concentration in the range of between about 0.05 and 0.5 percent by weight, based on the total weight of said ingestible product.

6. An ingestible product in accordance with any of Claims 1-3 wherein said sweetness inhibitor is present in a concentration in the range of between about 0.1 and 0.5 percent by weight, based on the total weight of said ingestible product.

7. An ingestible product in accordance with any of Claims 1-3 wherein said sweetener is selected from the group consisting of sucrose, fructose, corn syrup solids, high fructose corn syrup, dextrose, xylitol, sorbitol, mannitol, acetosulfam, dihydrochalcone, hydrogenated glucose syrups, aspartame, glycyrrhizin, stevioside and mixtures thereof.

8. An ingestible product in accordance with any of Claims 1-3 wherein said ingestible composition is a foodstuff selected from the group consisting of beverages, infused foods, sauces, condiments, salad dressings, fruit juices, syrups, desserts, icings and fillings, soft frozen products, confections, chewing gum and intermediate food.

9. An ingestible product in accordance with any of Claims 1-3 wherein said ingestible composition is a pharmaceutical preparation selected from the group consisting of toothpaste and mouthwash.

10. A process for inhibiting sweetness in an ingestible product comprising adding, to an ingestible composition containing a sweetener, an effective sweetness inhibiting amount of a compound selected from the group consisting of an alkali metal hexyl sulfate, an alkaline earth metal hexyl sulfate, an alkali metal heptyl sulfate, an alkaline earth metal heptyl sulfate and mixtures thereof.

11. A process in accordance with Claim 10 wherein said compound is selected from the group consisting of sodium hexyl sulfate, potassium hexyl sulfate, calcium hexyl sulfate, magnesium hexyl sulfate, lithium hexyl sulfate, sodium heptyl sulfate, potassium heptyl sulfate, calcium heptyl sulfate, magnesium heptyl sulfate, lithium heptyl sulfate and mixtures thereof.

12. A process in accordance with any of Claims 10-11 wherein said compound is selected from the group consisting of sodium hexyl sulfate, potassium hexyl sulfate, sodium heptyl sulfate, potassium heptyl sulfate and mixtures thereof.

13. A process in accordance with any of Claims 10-12 wherein said sweetness inhibiting compound is present in a concentration in the range of between about 0.005 and 1.0 percent by weight, based on the total weight of the ingestible product.

14. A process in accordance with any of Claims 10-13 wherein said compound is present in a concentration in the range of between about 0.05 and 0.5 percent by weight, based on the total weight of the ingestible product.

15. A process in accordance with any of Claims 10-12 wherein said compound is present in a concentration in the range of between 0.1 and 0.5 percent by weight, based on the total weight of the ingestible product.

16. A process in accordance with any of Claims 10-12 wherein said sweetener is selected from the group consisting of sucrose, fructose, corn syrup solids, high fructose corn syrup, dextrose, xylitol, sorbitol, mannitol, acetosulfam, thaumatin, invert sugar, saccharin, cyclamate, dihydrochalcone, hydrogenated glucose syrups, aspartame, glycyrrhizin, stevioside and mixtures thereof.

17. A process in accordance with any of Claims 10-12 wherein said ingestible composition is a foodstuff selected from the group consisting of beverages, infused foods, sauces, condiments, salad dressings, fruit juices, syrups, desserts, icings and fillings, soft frozen products, confections, chewing gum and intermediate foods.

18. A process in accordance with any of Claims 10-12 wherein said ingestible composition is a pharmaceutical preparation selected from the group consisting of toothpaste and mouthwash.

19. Use of a sweetness inhibitor selected from the group consisting of an alkali metal hexyl sulfate, an alkali earth metal heptyl sulfate, an alkaline earth metal heptyl sulfate and mixtures thereof for the preparation of an ingestible product.

Claims for Austria

1. A process for inhibiting sweetness in an ingestible product comprising adding, to an ingestible composition containing a sweetener, an effective sweetness inhibiting amount of a compound selected from the group consisting of an alkali metal hexyl sulfate, an alkaline earth metal hexyl sulfate, an alkali metal heptyl sulfate, an alkaline earth metal heptyl sulfate and mixtures thereof.

2. A process in accordance with Claim 1 wherein said compound is selected from the group consisting of sodium hexyl sulfat, potassium hexyl sulfate, calcium hexyl sulfate, magnesium hexyl sulfate, lithium hexyl sulfate, sodium heptyl sulfate, potassium heptyl sulfate, calcium heptyl sulfate, magnesium heptyl sulfate, lithium heptyl sulfate and mixtures thereof.

3. A process in accordance with any of Claims 1-2 wherein said compound is selected from the group consisting of sodium hexyl sulfate, potassium hexyl sulfate, sodium heptyl sulfate, potassium heptyl sulfate and mixtures thereof.

4. A process in accordance with any of Claims 1-3 wherein said sweetness inhibiting compound is present in a concentration in the range of between about 0.005 and 1.0 percent by weight, based on the total weight of the ingestible product.

5. A process in accordance with any of Claims 1-4 wherein said compound is present in a concentration in the range of between about 0.05 and 0.5 percent by weight, based on the total weight of the ingestible product.

6. A process in accordance with any of Claims 1-3 wherein said compound is present in a concentration in the range of between 0.1 and 0.5 percent by weight, based on the total weight of the ingestible product.

7. A process in accordance with any of Claims 1-3 wherein said sweetener is selected from the group consisting of sucrose, fructose, corn syrup solids, high fructose corn syrup, dextrose, xylitol, sorbitol, mannitol, acetosulfam, thaumatin, invert sugar, saccharin, cyclamate, dihydrochalcone, hydrogenated glucose syrups, aspartame, glycyrrhizin, stevioside and mixtures thereof.

8. A process in accordance with any of Claims 1-3 wherein said ingestible composition is a foodstuff selected from the group consisting of beverages, infused foods, sauces, condiments, salad dressings, fruit juices, syrups, desserts, icings and fillings, soft frozen products, confections, chewing gum and intermediate foods.

9. A process in accordance with any of Claims 1-3 wherein said ingestible composition is a pharmaceutical preparation selected from the group consisting of toothpaste and mouthwash.

10. Use of a sweetness inhibitor selected from the group consisting of an alkali metal hexyl sulfate, an alkali earth metal heptyl sulfate, an alkaline earth metal heptyl sulfate and mixtures thereof for the preparation of an ingestible product.

## DOCUMENTS CONSIDERED TO BE RELEVANT

| Category | Citation of document with indication. where appropriate, of relevant passages | Relevant to claim | CLASSIFICATION OF THE APPLICATION (Int. Cl.4) |
|---|---|---|---|
| A | EP-A-0 125 049 (TATE & LYLE PUBLIC LTD. CO.) * claim 1, examples 1-6 * | . | A 23 L 1/236<br>A 23 G 3/30<br>A 61 K 7/16 |
| | --- | | |
| A | DE-A-2 556 109 (GENERAL FOODS CORP.) * page 4, paragraph 1, claims 1, 13, 17, 18 * | | |
| | --- | | |
| P,A | EP-A-0 156 317 (GENERAL FOODS CORP.) * claims 1-4, 8 * | | |
| | ----- | | |

|  |
|---|
| TECHNICAL FIELDS SEARCHED (Int. Cl.4) |
| A 23 L 1/00<br>A 23 G 3/00<br>A 61 K 7/00 |

The present search report has been drawn up for all claims

| Place of search | Date of completion of the search | Examiner |
|---|---|---|
| BERLIN | 04-09-1986 | SCHULTZE D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82